# EUROPEAN PATENT SPECIFICATION

(11) **EP 3 398 438 B1**
(45) Date of publication and mention of the grant of the patent: **27.10.2021**
(21) Application number: 17169721.2
(22) Date of filing: 05.05.2017
(51) Int. Cl.: A21D 2/36, A21D 8/04, C12N 9/26

(54) **A BREAD WITH EXTENDED FRESHNESS**
BROT MIT VERLÄNGERTER HALTBARKEIT
PAIN À LA FRAICHEUR PROLONGÉE

(43) Date of publication of application: 07.11.2018
(73) Proprietor: Lantmännen Schulstad A/S, 2650 Hvidovre (DK)
(72) Inventor: Gerner Möller, Rune, 3480 Fredensborg (DK)
(74) Representative: Plougmann Vingtoft a/s

(56) References cited:
- EP-A1- 0 419 907
- WO-A1-99/53769
- WO-A1-2016/020478
- US-A- 2 615 810
- SILVERSTEIN O: "HEAT-STABLE BACTERIAL ALPHA-AMYLASE IN BAKING APPLICATION TO WHITE BREAD", BAKER'S DIGEST, SIEBEL PUBLISHING CO. PONTIAC, ILLINOIS, US, vol. 38, no. 4, 1 August 1964 (1964-08-01) , pages 66-70,72, XP001148912, ISSN: 0191-6114
- DATABASE GNPD [Online] MINTEL; June 2009 (2009-06), Anonymous: "Light Multi-Cereals Bread", XP002770980, Database accession no. 1125457
- "Enzymes solve baking, brewing problems", CHILTON'S FOOD ENGINEERING INTERNATIO,, vol. 16, no. 3, 1 January 1991 (1991-01-01), page 19, 22, XP009119145, ISSN: 0148-4478
- K Kamaljit ET AL: "Analysis of Ingredients, Functionality, Formulation Optimization and Shelf Life Evaluation of High Fiber Bread", American Journal of Food Technology, 1 April 2011 (2011-04-01), pages 306-313, XP055337091, DOI: 10.3923/ajft.2011.306.313 Retrieved from the Internet: URL:http://docsdrive.com/pdfs/academicjour nals/ajft/2011/306-313.pdf [retrieved on 2017-01-19]

## Description

### Technical field of the invention

The present invention relates to a bread with extended freshness. In particular, the present invention relates to a bread comprising wheat flour, heat-stable alpha amylase and oats, which displays an extended freshness/delayed staling.

### Background of the invention

Bread is a staple in diets all over the world. However, as much as 30% of the industrial bread consumers buy ends up being discarded. A major reason is that the consumers no longer perceive the bread as being fresh, despite it being nutritionally sound. Further, large volumes of bread are discarded by retailers, as they are aware of the consumer's bias.

This waste is unsatisfactory from both an economical and an environmental point of view. Providing a bread which has extended freshness and/or delayed staling would be advantageous.

Furthermore, there is a growing demand from consumers for organic and more natural products. There is a challenge in providing organic breads, which have a minimum of additives and preferably none that must be declared, and have extended freshness. Many of the additives typically used to extend freshness in bread are not suitable for inclusion in breads marked as organic, either because they are prohibited by legislation or because of consumer concern.

Hence, a bread with extended freshness would be advantageous, and in particular a bread which is possible to produce with organic ingredients, and with a minimum or no such problematic additives.

The problem of bread staling has long been the object of study and there are several solutions in the art.

WO9005443 relates to bread which does not stale. Breads described therein contains a milled groat, a high gluten wheat flour and guar gum. The breads also contain hydrophilic colloid, such as guar gum. However, the document states that alpha-amylases are not useful in baking bread commercially.

WO2007/056802 describes a white bread comprising wheat flour and a hydrophilic colloid such as guar gum.

WO 99/53769 describes the preparation of dough and baked products prepared from the dough, comprising incorporating into the dough an anti-staling amylase and a phospholipase. WO 99/53769 discloses that the anti-staling amylase has optimum activity at 70-90°C and phospholipase has optimum activity at 30-70°C.

However, there remains an unmet need for a white bread with extended freshness, and in particular a bread which may be produced with organic ingredients.

### Summary of the invention

Thus, it is an object of the present invention to provide a bread that solves the above mentioned problems of the prior art with staleness.

Thus, one aspect of the invention relates to a bread comprising
a) wheat flour,
b) at least one heat-stable alpha-amylase, wherein the heat-stable alpha-amylase when having an activity of 800 KNU/g is present in the range of from 1 to 3 ppm/kg flour, and
c) oats in an amount in the range of from 50 to 250 g per kg flour, and
d) at least one further non-starch hydrocolloidal plant polysaccharide selected from the group consisting of psyllium, chia and combinations thereof.

Another aspect of the present invention relates to a method of preparing a bread according to the invention comprising the steps of
i) providing wheat flour, at least one heat-stable alpha-amylase, wherein the heat-stable alpha-amylase when having an activity of 800 KNU/g is present in the range of from 1 to 3 ppm/kg flour, oats in an amount of from 50 to 250 g per kg flour, and psyllium and/or chia,
ii) forming a dough comprising the ingredients from step i), and
iii) baking said dough from step ii) to form a bread.

### Brief description of the figures

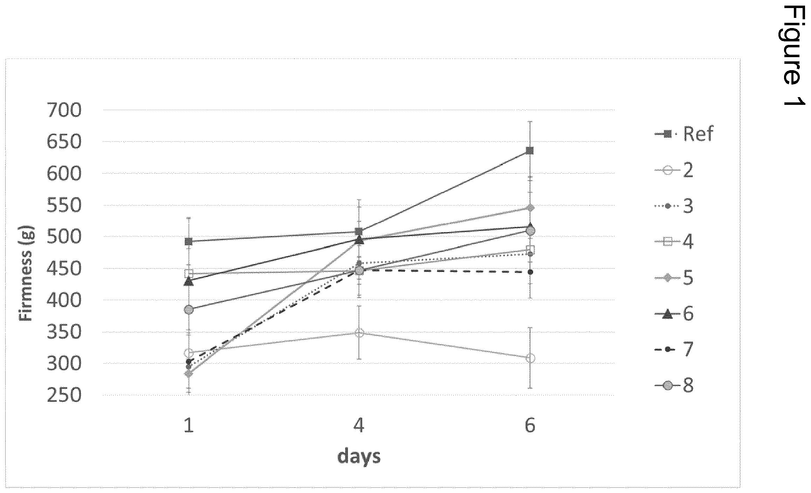

Figure 1 shows the results of texture analysis over time. See Example 2. Legend: x-axis: Days after baking; y-axis: Sample no.s 1 (Reference) to 8.

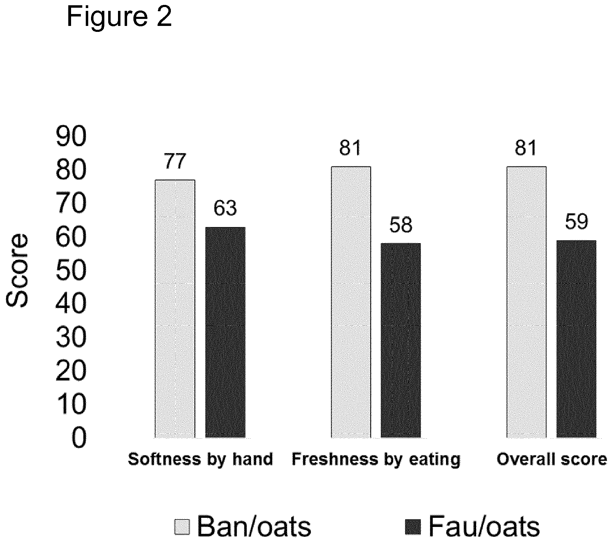

Figure 2 shows the results of sensory analysis of bread at Day 6. See further Example 3. Legend: y-axis: Score (Arbitrary units); x-axis: Softness by hand, Freshness by eating, Overall score. Light grey columns represent Ban/oats, i.e. samples comprising heat-stable alpha amylase (Ban) and oats. Black columns represent Fau/oats, i.e. samples comprising Fungamyl (non-heat-stable alpha amylase) and oats. It is seen that the Ban/oats samples receive higher scores than Fau/oats in all cases.

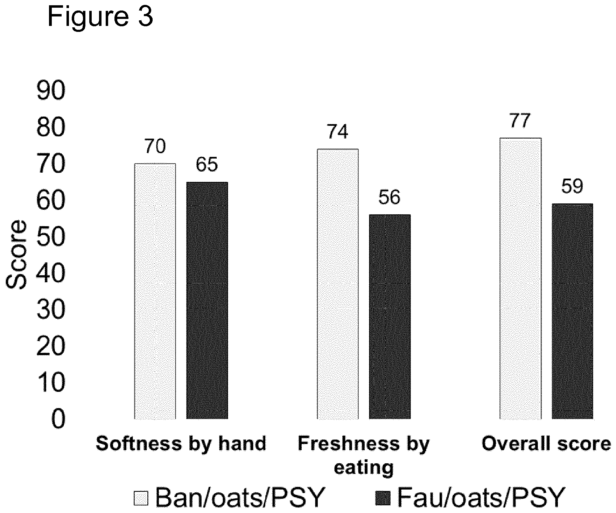

Figure 3 shows the results of sensory analysis of bread at Day 6. See further Example 3. Legend: y-axis: Score (Arbitrary units); x-axis: Softness by hand, Freshness by eating, Overall score. Light grey columns represent Ban/oats/PSY, i.e. samples comprising heat-stable alpha amylase (Ban), oats and psyllium. Black columns represent Fau/oats/PSY, i.e. samples comprising Fungamyl (non-heat-stable alpha amylase), oats and psyllium. It is seen that the Ban/oats/PSY samples receive higher scores than Fau/oats/PSY in all cases.

The present invention will now be described in more detail in the following.

### Detailed description of the invention

### Definitions

Prior to discussing the present invention in further details, the following terms and conventions will first be defined:
In the context of the present invention, mentioned percentages are weight/weight percentages unless otherwise stated.

The term "and/or" used in the context of the "X and/or Y" should be interpreted as "X", or "Y", or "X and Y".

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 1 to 8, from 3 to 7, from 4 to 9, from 3.6 to 4.6, from 3.5 to 9.9, and so forth.

All references to singular characteristics or limitations of the present invention shall include the corresponding plural characteristic or limitation, and vice versa, unless otherwise specified or clearly implied to the contrary by the context in which the reference is made.

Unless defined otherwise, all technical and scientific terms used herein have the same meaning as commonly understood by one of ordinary skill in the art.

### Description

The invention in a first aspect relates to a bread comprising
a) wheat flour
b) at least one heat-stable alpha-amylase, wherein the heat-stable alpha-amylase when having an activity of 800 KNU/g is present in the range of from 1 to 3 ppm/kg flour, and
c) oats in an amount in the range of from 50 to 250 g per kg flour, and
d) at least one further non-starch hydrocolloidal plant polysaccharide selected from the group consisting of psyllium, chia and combinations thereof.

### Bread

The bread according to the present invention is a wheat bread. The bread according to the present invention has extended freshness and/or delayed staling. In other words, the bread displays a long-lasting soft, moist feel and good resilience, also several days (for example 6 days, 7 days, 8 days, 9 days ) after baking. By good resilience is meant that the bread springs quickly back into shape after squeezing.

In one embodiment, the bread according to the present invention is essentially free from inclusions of intact or crushed grains or nuts, which would give the sensation of crunchy, or chewy. Instead, the bread is plain and soft, typically referred to as a white bread. The bread according to the present invention may be a sandwich bread and/or a toast bread.

The term "bread" as used herein also includes buns and bread rolls. A bun or bread roll may be defined as a bread baked in a smaller size, corresponding to single serving. The terms "buns" and "bread rolls" are used interchangeably.

The bread of the invention may be defined as leavened breads, such as yeast-leavened breads, such as a leavened white wheat bread. This is in contrast to flatbreads, such as tortilla breads and the like. Flatbreads typically have a very short baking time, such as a baking time in the range from 1 to 3 minutes. In one embodiment, the bread of the invention is not a flatbread.

The bread of the invention may be sweetened, for example with sugar, and may contain spices.

Thus, the invention in one embodiment relates to a bread according to the present invention, wherein said bread is a leavened white wheat bread. A further embodiment relates to a bread according to the present invention, wherein said bread is not a flatbread.

In particular embodiments, the invention relates to a bread according to the present invention wherein said bread is a bread roll.

### Heat stable alpha amylase

The bread according to the present invention comprises at least one heat-stable alpha amylase.

Alpha-amylases (EC number 3.2.1.1) are enzymes which hydrolyze (1,4)-alpha-D-glucosidic linkages in starch polysaccharides, yielding glucose and maltose. They are endo-active enzymes, that is they preferably cleave linkages inside the starch polysaccharide, as opposed to exoenzymes, which cleave off terminal groups from the ends.

Heat stable alpha-amylases retain their activity despite high temperatures. Normal, non-heat stable alpha amylases function best in the range of 30 to 40 °C, and are usually inactivated or destroyed at temperatures above 45 °C.. Heat stable variants are able to be active, i.e., catalyse bond cleavage, at temperatures above 45 °C. For example, an alpha amylase may be defined as heat stable if it displays, in *in vitro* test systems, at least 90% of its activity after being incubated for 60 minutes at pH 6.2, at 85 °C. In other embodiments, an alpha amylase may be defined as heat stable if it displays at least 92 %, such as at least 95%, or at least 96%, such as at least 97%, or for example at least 98% activity after being incubated for 60 minutes at pH 6.2, at 85 °C. Heat stable alpha amylases are also referred to as thermostable alpha amylases.

Tests for alpha amylase activity are well known in the art. See for example test described on Sigma Aldrich, or in Bernfeld, P. (1955) Methods in Enzymology 1, 149-158. Further methods are published in the series Methods in Enzymology, and the selection and application of methods is within the skill of a person skilled in the art.

Non-heat-stable alpha amylases are used in bread making in order to, *inter alia* improve brown crust colour, ensure fine and uniform crumb structure, and/or increase loaf volume. They are not used in order to delay staling (prolong softness). Non- heat-stable alpha amylases are discussed further under the heading "Further enzymes", *q.v.*

The inventors have surprisingly realised the particular effect of heat-stable alpha amylase, which effect is to extend softness and/or delay staling. The term extend softness" means extending the amount of time that the bread has an acceptable softness. In other words, the bread remains soft for longer period of time, as compared to a conventional comparable bread. Heat-stable alpha amylases are typically used for enhanced liquefaction, for example in beverages such as alcohol, and therefore the softening effect of heat-stable alpha amylases in bread, such as in breads according to the present invention, is surprising.

For example, BAN 800, a heat-stable alpha amylase, is not marketed for baking dough, but instead for enhanced liquefaction.

There are several heat-stable alpha amylases available commercially which are suitable for the invention. In some particular embodiments, the invention relates to a bread according to the present invention wherein the heat-stable alpha amylase is selected from the group consisting of Max life U4 Bac (DuPont), BakeZyme AN 301 Bac (DSM), AmyloFresh (Veron), BAN 800 BG ® (Novozymes), Termamyl (Novozymes), and/or Liquizyme ® (Novozymes).

In one specific embodiment, the heat-stable alpha amylase is an enzyme used in liquefaction, such as BAN 800 BG ®, Termamyl ® and/or Liquizyme ® all from Novozymes.

One example of bacterial heat-stable alpha amylase useful in the present invention is Bacterial alpha amylase Ban 800 BG ® by Novozymes. This enzyme is produced in *Bacillus amyloliquefaciens,* and is classified as a non-GMO enzyme.

Some customers express concern over the safety of genetically modified organisms, in particular in foods. There is a demand for foods which are free from GMO-products.

Thus, in one particular embodiment the invention relates to a bread according to the present invention wherein the heat-stable alpha amylase is non-GMO enzyme.

The bread according to the present invention may further comprise other enzymes which contribute to softness. In a particular embodiment relates to the bread according to the present invention wherein the bread further comprises at least one xylanase.

In one embodiment of the bread according to the present invention, no further enzymes beyond the heat-stable alpha amylase are added and/or are present.

The amount of heat-stable alpha amylase included is based on the amount of wheat flour present in the bread of the invention.

Thus, the amount of heat-stable alpha amylase, for example the amount of a heat-stable alpha amylase having an activity of 800 KNU/g, present in the bread according to the present invention may be in the range of from 1 to 3 ppm/kg flour, such as from 1.0 to 2 ppm/kg flour; or for example from 1.0 to 1.9 ppm/kg flour, such as from 1.0 to 1.8 ppm/kg flour, for example from 1.0 to 1.7 ppm/kg flour, such as from 1.0 to 1.6 ppm/kg flour, for example from 1.0 to 1.5 ppm/kg flour, such as from 1.0 to 1.4 ppm/kg flour, for example from 1.0 to 1.3 ppm/kg flour, such as from 1.0 to 1.2 ppm/kg flour.

The determination of the activity of the heat-stable alpha amylase is within the scope of a person skilled in the art.

### Further enzymes

In some embodiments, the bread according to the present invention further comprises at least one non-heat stable alpha amylase and/or at least one xylanase.

As used herein, the term "non heat-stable alpha amylase" is used to indicate alpha amylases with normal sensitivity to heat, i.e. alpha amylases that function best in the range of 30 to 40 °C, and that are usually inactivated or destroyed at temperatures above 45 °C.

Said at least one non -heat-stable alpha amylase may be for example a fungal alpha amylase, a bacterial alpha amylase, or combinations thereof. Examples of said at least one non heat-stable alpha amylase include Fungamyl ® from Novozymes.

Said at least one xylanase may be for example a fungal xylanase, a bacterial xylanase, or combinations thereof. Examples of said at least one xylanase include Panzea ® from Novozymes and Pentopan ® from Novozymes.

Xylanases are used in order to improve dough properties such as elasticity and stability.

Selection of the amounts of the mentioned enzymes which may be further comprised, is within the skill of the person skilled in the art. He or she is referred to the product sheet(s) for the particular enzyme.

In specific embodiments, the bread according to the present invention further comprises a non-heat-stable alpha amylase (such as Fungamyl), in an amount in the range of from 1 U/kg flour to 10 U/kg flour, such as from 2 U/kg flour to 8 U/kg flour, for example from 4 U/kg flour to 8 U/kg flour.

In further specific embodiments, the bread according to the present invention further comprises a xylanase (such as Pentopan and/or Panze), in an amount in the range of from 50 to 120 U/kg flour, such as from 60 U/kg flour to 100 U/kg flour, for example from 60 U/kg flour to 90 U/kg flour.

### Excluded ingredients

There are several known ingredients which may be included in a bread in order to extend freshness of bread. However, many of these are problematic, as they may not be allowed to be included in goods marked organic, and/or are not considered natural or healthy by the consumer. The bread according to the present invention can remain fresh without addition of such problematic additives such as parabens, sulphites, trans-fats, high fructose corn syrup, and polysorbate.

Thus, in one embodiment, the bread according to the present invention does not comprise parabens, sulphites, trans-fats, high fructose corn syrup, nor polysorbate.

### Additives

The bread according to the present invention may comprise at least one additive selected from the group consisting of calcium carbonate, lactic acid, carbon dioxide, malic acid, ascorbic acid, formic acid, nitrites, tocepherols, lecithins, citric acid, calcium citrate, L-tartaric acid, sodium tartrate, potassium tartrate, monocalcium phosphate, alginic acid, acetic acid (vinegar), sodium alginate, potassium alginate, agar, glycerol, pectin, sodium carbonates, potassium carbonates, ammonium carbonates, magnesium carbonate, calcium sulphate, sodium hydroxide, silica dioxide and combinations thereof. The additives of said group are considered natural additives for the purposes of this invention. Additives from said group may be added to foods that are marked Ecological or Organic.

Particular embodiments relates to a bread according to the present invention further comprising at least one additive selected from the group consisting of ascorbic acid, lactic acid and/or calcium citrate.

Further particular embodiments relates to a bread according to the present invention further comprising ascorbic acid, for example in an amount in the range of from 20 to 120 U/kg flour, such as from 30 to 120 U/kg flour.

Yet further particular embodiments relates to a bread according to the present invention further comprising ascorbic acid (for example in amount as described above), as well as at least one non-heat stable alpha amylase and/or at least one xylanase (see further discussion under heading "Further enzymes".

### Wheat flour

The bread according to the present invention comprises wheat flour. The wheat flour may be any suitable wheat flour, for example one or more selected from the group consisting of all-purpose flour, bread flour, German type 550 flour, Reform flour, Manitoba flour, durum wheat flour and combinations thereof. See further discussion under the heading "Gluten" regarding gluten content of flour.

Herein, the term flour is understood to refer to wheat flour, unless explicitly stated otherwise.

There are generally speaking four types of wheat flour. White flour is made from the endosperm only of the wheat grain. Brown flour includes some of the some of the grain's germ and bran, while whole grain or wholemeal flour is made from the entire grain, including the bran, endosperm, and germ. Germ flour is made from the endosperm and germ, excluding the bran.

Thus, in some embodiments, the invention relates to the bread according to the invention, wherein the wheat flour is selected from the group consisting of white flour, brown flour, wholemeal flour, germ flour and combinations thereof.

In one particular embodiment, the invention relates to a bread according to the present invention wherein the wheat flour comprises or consists of white flour and/or whole wheat flour.

In a further particular embodiment, the invention relates to a bread according to the present invention wherein the wheat flour consists of white flour.

### Oats

The bread according to the present invention comprises oats. By the term "oats" is meant the berries of the plant *Avena sativa.* Oats are a good source of dietary fibre, beta glucans, vitamins and minerals, and also impart a good taste.

The oats that are used in the bread are typically not the unprocessed oat berries (also known as groats). Instead the oat berries are de-husked, steamed and rolled into flat flakes. Oat berries processed in this manner are referred to herein as "rolled oats". Thus, in one embodiment the invention relates to a bread according to the present invention wherein the oats comprise rolled oats. Further embodiments relate to a bread according to the present invention wherein the oats are not groats, for example where the oats consist of rolled oats.

While oats have the advantage of binding water and thereby improving freshness (delaying staling), inclusion of oats can lead to stickiness, which is an undesirable texture in bread. Including oats can sometimes have a negative effect on texture by increasing crumbliness.

The inventors have observed that the form in which the oats are provided influences the water-binding capacity of oats, and thereby also influences the impact of the oats on the texture of the bread. For instance, providing the oats as oat flour yields a product which has increased stickiness. However, providing the oats in form of intact and/or cut rolled oats yields breads with improved softness without tooth pickiness, and without stickiness.

Stickiness may be also be overcome by including rough rolled oats in the bread of the invention. The particle size of oats is typically given by measure of weight per volume (gram per liter, g/L). The term "rough rolled oats", as used herein, refers to rolled oats which have a weight by volume in the range of from 360 g/L to 450 g/L, such as 370 g/L to 430 g/L. Breads which comprise rough rolled oats, i.e. rolled oats with a weight by volume in this range show a good effect on bread texture, i.e. display a reduced stickiness and extended freshness and/or delayed staling.

Thus, one particular embodiment relates to a bread according to the present invention wherein the oats as provided comprise rough rolled oats as described above.

The unpleasant texture which may arise on inclusion of oats in the bread, may alternatively be avoided by providing the oats for the bread as a mixture of intact rolled oats and cut rolled oats. The intact rolled oats may be in the form of rough rolled oats, as discussed above.

A further particular embodiment of the invention relates to a bread according to the present invention which comprises oats (for example rolled oats), which are provided not as intact rolled oats, but instead as cut rolled oats.

Cut rolled oats are commercially available and are typically described by flake size. In one embodiment the oats in the bread according to the present invention are of a size in the range of from 0.4 mm to 1.2 mm, or for example 0.5 to 1.0 mm, such as from 0.8 to 1.0 mm.

In a further particular embodiment, the invention relates to a bread according to the present invention wherein the oats for said bread consist of cut rolled oats.

In an aspect, the bread according to the present invention relates to the amount of oats present, calculated as g per kg flour (see Example 1 for example of calculation), is in the range of from 50 to 250 g oats per kg flour, such as in from 50 to 200 g oats per kg flour, for example 50 to 175 g oats per kg flour, such as 50 to 160 g oats per kg flour, for example 50 to 155 g oats per kg flour, such as 50 to 150 g oats per kg flour; such as 55 to 250 g oats per kg flour, 60 to 200 g oats per kg flour, 75 to 200 g oats per kg flour, or for example 75 to 180 g oats per kg flour, such as 80 to 160 g oats per kg flour.

In preferred embodiments, even though the oats are provided as, for example, intact rolled oats, the baking process leads to the disintegration of the oats and in the final bread no intact oats or pieces of oats that are observable by eye are present.

### Non-starch hydrocolloidal plant polysaccharide

Beta-glucans present in oats may be defined as a non-starch hydrocolloidal plant polysaccharide.

Thus, an aspect of the invention relates to bread according to the present invention comprising at least one further non-starch hydrocolloidal polysaccharide.

The term "non-starch hydrocolloidal plant polysaccharide" as used herein refers to non-starch polysaccharides from plants (including marine plants), which have gelling qualities. These hydrocolloidal polysaccharides are not starch molecules. They are able to bind many times their weight in water, and they contribute to improving freshness by binding moisture in the bread. The beta-glucans present in oats are one example of non-starch hydrocolloidal plant polysaccharides.

Inclusion of non-starch hydrocolloidal plant polysaccharides in bread can negatively influence the texture of a bread, leading to stickiness and/or also to reduced "bite", crumbliness, etc. However, the present inventors have noted that by including both oats as described above (see heading Oats), and at least one further non-starch hydrocolloidal plant polysaccharide, the negative influence of both the oats and the non-starch hydrocolloidal plant polysaccharides can be overcome, resulting in an extended freshness (delayed staling) with minimal negative effect on the texture.

In an aspect, the at least one further non-starch hydrocolloidal plant polysaccharide comprises psyllium and/or chia. In a further particular embodiment, the at least one further non-starch hydrocolloidal plant polysaccharides consists of psyllium and/or chia.

The term "psyllium", as used herein refers to a preparation of the psyllium seed husks comprising the hydrocolloidal plant polysaccharide. Psyllium is also known as ispaghula. Psyllium may be derived from the plant *Plantago ovata.* In a particular embodiment, the psyllium in the bread according to the present invention is in the form of milled psyllium husks.

The term "chia" as used herein refers to the preparation of the seeds from the plant *Salvia hispanica* comprising the non-starch hydrocolloidal plant polysaccharides. In a particular embodiment, the chia in bread according to the present invention is in the form of milled chia seeds.

In particular embodiments of the bread according to the present invention, the non-starch hydrocolloidal plant polysaccharide is present in amount in the range of from 5 to 32% of total weight of ingredients (see Example 1 for example of calculation), for example in the range of from 7% to 30%, from 9% to 30%, such as from 10% to 30%, from 11% to 30%, from 12% to 30%, such as from 13% to 30%, for example from 14% to 30%, 15 to 30%, for example from 16 to 30%, such as from 17 to 30%, for example from 18 to 30%, for example from 19 to 30%, such as from 20 to 30%; or for example in the range of from 5 to 30%, for example from 5 to 28%, such as from 5 to 27%, for example from 5 to 26%, such as from 5 to 25%, 5 to 24%, 5 to 23%, for example from 5 to 22%, such as from 5 to 21%, or for example from 5 to 20 %; or for example from 7 to 27%, 7 to 25%, or 8 to 25%, or for example from 9 to 25%, all percentages by weight of the total weight of the ingredients.

The inventors have surprisingly shown that breads prepared comprising wheat flour, oats, heat-stable alpha amylase, and psyllium are particularly soft and yet retain good bite. See for example Examples 2 and 3.

Thus, the bread according to the present invention relates to a bread comprising a) wheat flour, b) at least one heat-stable alpha-amylase, and c) oats, and at least one further non-starch hydrocolloidal plant polysaccharide, selected from the group consisting of psyllium and chia.

A further particular embodiment of the bread according to the present invention relates to a bread comprising a) wheat flour, b) at least one heat-stable alpha-amylase, and c) oats, further comprising at least one further non-starch hydrocolloidal plant polysaccharide selected from the group consisting of psyllium and chia, and further comprising at least one non-heat stable alpha amylase and/or at least one xylanase.

### Gluten

The texture of wheat bread is dependent on the gluten network in the bread. The term "gluten" is used herein as a collective term to designate the network-forming proteins present in wheat. Increasing the amount of gluten in bread leads to stronger and/or more gluten networks being formed in the bread, which in turn influence the bread's texture. The amount of gluten present in bread can be increased by using wheat flour with relatively high amounts of gluten, so called "strong" wheat flour. Such wheat flours typically have a gluten content in the range of from 8- 14% w/w by weight.

Thus, in one embodiment the invention relates to a bread according to the present invention wherein the wheat flour is a strong wheat flour, such as a wheat flour with a gluten content in the range of from 8-14 % gluten, such as 8 to 12% gluten, such as from 8 to 9%; or for example from 10 to 13% gluten.

The amount of gluten present may also be enhanced by adding additional gluten protein to the bread. Thus, one embodiment relates to a bread according to the present invention, wherein said bread further comprises additional gluten, in addition to the gluten provided by the wheat flour.

Such additional gluten may be provided in the form of powdered gluten protein. Such preparations are available commercially. In particular embodiments the invention relates to a bread according to the present invention comprising an amount of additional gluten (i.e., in addition to the gluten provided by the wheat flour) in the range of from 0.5 % to 5%, based on weight of flour, such as for example 0.5% to 4%, 0.5% to 3%, 0.5% to 2%g gluten based on the weight of flour; or for example an amount in the range of from 0.5% to 5%, 0.8% to 5% gluten based on the weight of flour.

Thus, a further particular embodiment of the bread according to the present invention relates to a bread comprising a) wheat flour, b) at least one heat-stable alpha-amylase, and c) oats, further comprising at least one further non-starch hydrocolloidal plant polysaccharide, such as a non-starch hydrocolloidal plant polysaccharide selected from the group consisting of psyllium and chia; and further comprising at least one non-heat stable alpha amylase and/or at least one xylanase; wherein said bread further comprises additional gluten, in addition to the gluten provided by the wheat flour, in an amount in the range from 0.5 % to 5%, based on weight of flour.

### Organic bread

The ingredients in the bread according to the present invention may be obtained as organic versions and therefore the invention in one particular embodiment relates to a bread according to the present invention, being an organic bread.

The term "organic" as used herein refers to an item that is certified organic. The terms "organic" and "ecological" are used interchangeably herein. The certification aims to verify that the product does minimal negative impact on nature. The certification of goods as being "organic" or "ecological" is typically regulated by national legislative bodies, and requirements vary between countries. The regulation for the EU is regulated in Council Regulation (EC) No 834/2007 of 28 June 2007 on organic production and labelling of organic products and repealing Regulation (EEC) No 2092/91. Thus, one embodiment of the invention relates to a bread according to the present invention wherein the bread fulfils the requirements for being certified organic according to the EU legislation.

A further embodiment relates to a bread of according to the present invention which fulfils the requirements for being certified organic according to Danish Law.

A further embodiment relates to a bread according to the present invention wherein a maximum of 5% non-organic ingredients is present.

### Further ingredients

In some embodiments, the bread according to the present invention further comprises one or more further grains, such as for example one or more selected from the group consisting of rye, barley and spelt.

### Process and products obtainable bv the process

In another the aspect the invention relates to a method of preparing a bread according to the invention comprising the steps of
i) providing wheat flour, at least one heat-stable alpha-amylase, wherein the heat-stable alpha-amylase when having an activity of 800 KNU/g is present in the range of from 1 to 3 ppm/kg flour, oats in an amount of from 50 to 250 g per kg flour, and psyllium and/or chia,
ii) forming a dough comprising the ingredients from step i) and
iii) baking said dough from step ii) to form a bread.

In further embodiments of this aspect, the invention relates to a method according to the present invention, wherein said oats in step i) are selected from the group consisting of intact rough rolled oats, cut rough rolled oats, intact rolled oats, cut rolled oats, and combinations thereof. Oats useful in the invention are discussed elsewhere herein, see in particular discussion of the form in which oats are provided and influence on texture under the heading Oats.

In further embodiments of this aspect, the invention relates to a method according to the present invention, wherein said heat-stable alpha amylase in step i) is selected from the group consisting of Max life U4 Bac (DuPont), BakeZyme AN 301 Bac (DSM), AmyloFresh (Veron). Heat-stable alpha amylases useful in the invention are discussed elsewhere herein, see for example under the heading Heat-stable alpha amylases.

In further embodiments of this aspect, the invention relates to a method according to the present invention, wherein said wheat flour in step i) is selected from the group consisting of white flour, brown flour, wholemeal flour, germ flour and combinations thereof. Wheat flour useful in the invention are discussed elsewhere herein, see for example under the headings Wheat flour and Amount of gluten.

In further embodiments of this aspect, the invention relates to a method according to the present invention, wherein the baking of step iii) is a baking time for at least 4 minutes, such as for at least 5 minutes, for at least 6 minutes, for example at least 7 minutes; or for example from 4 to 25 minutes, 4 to 20 minutes, 4 to 15 minutes, 4 to 13 minutes, 4 to 12 minutes, 4 to 11 minutes, 4 to 10 minutes, 4 to 9 minutes, 4 to 8 minutes, 4 to 7 minutes; or for example from 5 to 25 minutes, 5 to 20 minutes, 6 to 20 minutes, 7 to 20 minutes, 8 to 20 minutes, 9 to 20 minutes; or for example for 4 to 25 minutes, 5 to 22 minutes, 6 to 19 minutes, such as 4 to 16 minutes or 4 to 18 minutes.

A baking time in the mentioned intervals are suitable for baking breads and/or buns of the present invention, such as leavened breads (for example yeast-leavened breads) of the invention. (See further discussion under heading "Bread").

In further embodiments of this aspect, the invention relates to a method wherein step ii) of forming a dough is by using the Chorleywood Bread Process (CBP). CBP is a method of baking bread which is well-known in the art.

### Further aspects

The method according to the present invention yields a dough in step ii) of the method. The dough in itself may be a product. The dough may be prepared for baking, but not actually baked. Instead the dough may be sold to the consumer, for baking in the consumer household. The dough may be further processed in order to be made suitable for such sale, such as being proofed and/or packaged for sale, and/or frozen.

### Examples

### Example 1

A bread was produced according to the straight dough method. The ingredients are listed in Table 1 below.

**Table 1: Ingredients in bread**

| Ingredient | Amount (g) |
|---|---|
| Organic Wheat flour | 368 |
| Organic whole wheat flour | 582 |
| Organic oats | 50 |
| Yeast | 50 |
| Salt | 15 |
| Organic sugar | 54 |
| Cardamom | 1 |
| Psyllium | 20 |
| Organic sunflower oil | 40 |
| Organic gluten | 40 |
| Organic Kimo* | 10 |
| Vinegar | 15 |
| Water | 695 |
| TOTAL | 1940 |

| | |
|---|---|
| *Kimo: dough improver comprising alpha amylase and xylanase | |

This yields a psyllium content of 20 /1940 = 0,010 = 1 % by weight of the total amount of ingredients. The amount of oats present is 50 /1940 = 0,025 = 2.6%.

Alternatively, the amounts of the dry ingredients may be given as a percentage of the weight of flour. Thus, the psyllium content of the recipe in Table 1 is (20 g/368 g) ^{∗} 1000 = 54 g psyllium per kg flour; and the oats content of the same recipe is (50 g/368g) ^{∗} 1000 = 135 g oats per kg flour.

Bread buns were prepared according the recipe in Table 1 using the straight dough method. All buns were baked on the same day (Day 0). After baking they were packed in clear plastic bags and stored at ca 20 °C

### Example 2- Texture evaluation

The texture was evaluated on days after baking by use of a Texture Analyzer. Texture analysis was performed on Day 1, Day 4, Day 6 and Day 8.

The samples were prepared according to Table 3.

Texture evaluation was done using a TVT Texture Analyzer (Perten Instruments), which measures both Firmness and Springiness. The method used is described in TVT Method 01-04-02. The probe used is P-CY 22S, and the program used was Crumb firmness and springiness (Hold until time compression).

**Table 2: Parameters of texture analyzer**

| Parameter: Hold until time compression | |
|---|---|
| Sample height (mm) | 25.0 |
| Starting distance from sample (mm) | 5.0 |
| Custom force or distance (mm) | 6.25 |
| Compression (mm) | 32.0 |
| Hold time (sec) | 32.0 |
| Data acquisition time (sec) | 30.0 |
| Initial speed (mm/sec) | 1.0 |
| Test speed (mm/sec) | 1.7 |
| Retract speed (mm/sec) | 10.0 |
| Trigger force (g) | 5 |
| Data rate (pps) | 250 |

Briefly, bread is sliced into 2.5 cm thick, and placed on the measuring table centered below the probe. The recording of the measurement data starts once the probe reaches the pre-set trigger force. The probe then compresses the sample to a pre-defined distance and hold in that position during a pre-set time. After compression, the probe returns to its starting position.

Springiness is a measure of how a product physically springs back after it has been deformed during the first compression. It is the ratio of the distance recorded during second compression of a sample to that recorded during its initial compression. If the value is 1, the product springs back completely.

The results of texture analysis is seen in Figure 1. Figure 1 shows the texture analysis in respect of Firmness (measured in g). The firmness is defined by this method as the force required to compress the bread sample to a certain distance. The larger the force required, the firmer the bread is. Typically, firmness increases over time indicating staling. The figure shows that all samples increase in firmness, except Sample 2, which actually displays an initial firming, and then a decrease in firmness between day 4 and day 6.

**Table 3: Samples**

| SAMPLE | Heat-stable alpha amylase (BAN 800) (ppm /kg flour) | Oats (% /kg flour) | Psyllium (%/kg flour) |
|---|---|---|---|
| 1 (Control) | | | |
| 2 | 1.5 | | |
| 3 | 1.5 | 5 | |
| 4 | 1.5 | | 2 |
| 5 | 1.5 | 5 | 2 |
| 6 | | 5 | |
| 7 | | | 2 |
| 8 | | 5 | 2 |

The breads were also subjected to a sensory analysis by a panel of testers. The results of the sensory analysis is given in Table 4. As can be seen from the table, even though sample 2 displayed the least firmness according to texture analyser, this sample does not have the most preferred texture by sensory analysis. It displays an almost too moist and sticky texture on days 1 and 4. Also, in contrast with the texture analyser, the texture of sample 3 is experienced as soft as sample 2, and sample 4 as even better than sample 2.

While objective measurements such as texture analysis is important, ultimately staleness is a subjective experience by the consumer. Thus, the sensory analysis gives important information that the bread according to the present invention actually provides a better experience of freshness than heat-stable alpha amylase alone.

**Table 4: Results of sensory analysis**

| | Day 1 | Day 4 | Day 6 | Day 8 Only touch evaluation |
|---|---|---|---|---|
| 1 (Ref) | Fells fresh and soft by hand, good bite | Still moist, however some decrease in softness and eating properties | Dry when touching bun, feels hard in mouth | Lack of freshness & moistness. Some mould on bun |
| 2 (1,5 ppm Ban) | Fells fresh and soft, tendency to sticky crumb. Tooth picking when eating | Fells moist, tendency to sticky crumb. Softer than Ref. | Still soft, better than Ref. Like day 4 on bread parameters | Softer than Ref, good after 8 days |
| 3 (1,5 ppm Ban- 5 % oats) | Fells fresh, Oats seems to reduce stickiness compare Ban. Feels good in mouth. More bite than Ban | Like day 1, but tendency to harder bite compare to day 1 | Still soft, no sticky crumb. Better texture than Ref & Ban | Softer than ref, like Ban |
| 4 (1,5 ppm Ban- 2 % PSY) | Very smooth and soft, no sticky crumb, no tooth picking | Very smooth and soft, no sticky crumb, no tooth picking | Very soft, good eating quality. Feels more moist than Ban & oats. No sticky crumb | Feels softer than ref & Ban / oats, but some hardness effect |
| 5 (1,5 ppm Ban- 2 % PSY-5 % oats) | Feels fresh and soft by hand, good bite, no stickiness on crumb. | Soft & fresh, firmer bite compare to Ban & oats. | Still moist and fresh, harder bite compare to Ban & PSY | Still soft, fells more soft than Ban alone |
| 6 (5 % oats) | Feels fresh, harder bite than Ref. | Like day 1,some hardness effect noted compare to Ban & oats | Feels hard by touch, not so fresh | Feels hard, similar to control |
| 7 (2 % PSY) | Feels fresh, smooth surfaces, good bite | Fells fresh, some hardened effect noted | Still soft and eatable, but not so soft as Ban/PSY/oats | feels softer than Ref, however not as soft compare to Ban/PSY & Oats |
| 8 (2 % PSY-5 % oats) | Feels fresh, harder bite than PSY alone | Feels fresh, however some hardness effect noted compare to day 1 | Softer than Ref, but some lack in freshness compare to Ban/PSY/oats | Feels softer than Ref, better than oats. |

### Example 3

The following example is to illustrate the difference between and a standard alpha amylase and a heat-stable alpha amylase.

The breads were prepared as described in Example 1. They were packed in clear plastic bags and stored at 20 °C for 6 days. The sensory analysis was performed on Day 6.

The analysis was performed by 12 untrained judges. Samples were presented on trays following international guidelines for sensory evaluation.

### Instructions to judges

Bread softness by hand: Use 2-3 fingers to compress the bread crumb. Evaluate the force needed to compress the crumb. More force indicates hard bread and is given a lower performance, less force indicates softer bread and is given a higher performance.

Freshness by eating: Take a bite of the bread and evaluate the amount of saliva needed to chew the bun. High amount of saliva indicates a dry bun crumb and is given a lower performance score. Low amount of saliva indicates a moist crumb and is given a higher performance score.

Overall- hand/mouth performance: The combination of hand and mouth performance. Low score indicates dry and poorer eating quality. High score indicates increased softness and better eating properties.

The panellists were asked to evaluate the buns on three parameters, listed in Table 5. They were asked whether they were able to taste to taste see or feel a difference between two different products. Softness by hand means whether how fresh the breads were experienced as being, upon touching/pressing. Freshness by eating means how fresh the breads were experienced as being upon tasting. This includes evaluation of crumb, stickiness, bite etc. Finally, the third parameter is a collective evaluation on whole, of how fresh the breads were considered to be.

The buns were scored from 1 to 10, where 10 indicates highest performance and 1 lowest. Simple statistical analysis was used to see the difference. The results are presented in Table 5 below, as well as shown in Figure 2.

### Sensory set up:

Test I: Sample 9 (BAN 800 MG/oats) vs Sample 10 (Fungal amylase fungamyl 2500 SG/oats)
Test II: Sample 11 (heat-stable alpha amylase BAN 800 MG/oats/psyllium) vs Sample 12 (Fungal amylase Fungamyl 2500 SG/ oats/ psyllium)

### Results:

Test I: The judges had a clear preference of the two tested buns with different enzymes. Bread buns with heat-stable alpha amylase (BAN 800 mg) scored higher on all parameters compared to Fungamyl 2500 SG. Thus, bread comprising heat-stable alpha amylase and oats are significantly better in these parameters compared to bread comprising non-heat-stable alpha amylase and oats.
Test II: The judges could easily discern the difference between the two samples. The breads comprising BAN/oat/psyllium were scored higher than fungal amylase/oats/psyllium.

The untrained panellists could easily pick up the difference between the two different breads with the different enzymes. Heat-stable alpha amylase was clearly preferred by the judges.

Thus, in conclusion the results show that breads comprising a heat-stable alpha amylase are experienced as more fresh and less stale, than those breads which are made using a non-heat-stable alpha amylase (Fungamyl ®, by Novozymes).

It also shows that the bread comprising oats and BAN (sample 9), was considered fresher than the bread further comprising Psyllium (sample 11).

**Table 5: Results of sensory analysis of heat-stable alpha amylase vs alpha amylase**

| | 9 (BAN/oats) | 10 (FUA/oats) | 11 (BAN/oats/PSY) | 12 (FUA/oats/PSY) |
|---|---|---|---|---|
| Softness by hand | 77 | 81 | 70 | 65 |
| Freshness by eating | 81 | 58 | 74 | 56 |
| Overall Score | 81 | 59 | 77 | 59 |

### Example 4

The following is an example of a bread according to the present invention.

**Table 6: Organic bread rolls**

| Ingredient | Amount % based on flour weight |
|---|---|
| Wheat flour (organic) | 58 |
| Wholewheat flour (organic) | 33.1 |
| Yeast mixture | 10 |
| Salt | 1.5 |
| Water | 54.8 |
| Wheat gluten (organic) | 1 |
| Oats | 7.9 |
| Sugar | 7 |
| Psyllium | 1.5 |
| Enzyme solution | 0.380 |
| Sunflower seed oil | 4 |
| Vinegar | 1.2 |

The dough is shaped to bread rolls and baked for 9 minutes.

### Example 5

A further example of the bread according to the present invention is given in Table 7. The dough is shaped to bread rolls and baked for 9 minutes.

**Table 7: Organic bread rolls, wholewheat**

| Ingredient | Amount % based on flour weight |
|---|---|
| Rye kernels, rolled | 15 |
| Water | 18.1 |
| Wheat flour | 45.1 |
| Sprouted rye | 7.6 |
| Yeast | 10 |
| Salt | 1.5 |
| Wholewheat flour | 22.6 |
| Water | 27 |
| Organic wheat gluten | 2 |
| Organic oats | 7.8 |
| Organic sugar | 5 |
| Organic Psyllium | 1.5 |
| Enzyme solution | 0.230 |
| Organic sunflower seed oil | 4 |
| Organic Vinegar | 1.5 |

## Claims

1. A bread comprising
a) wheat flour
b) at least one heat-stable alpha-amylase, wherein the heat-stable alpha-amylase when having an activity of 800 KNU/g is present in the range of from 1 to 3 ppm/kg flour, and
c) oats in an amount in the range of from 50 to 250 g per kg flour, and
d) at least one further non-starch hydrocolloidal plant polysaccharide selected from the group consisting of psyllium, chia and combinations thereof.

2. The bread according to claim 1, wherein said at least one further non-starch hydocolloidal plant polysaccharide is psyllium.

3. The bread according to any of the preceding claims wherein said bread further comprises at least one non-heat-stable alpha-amylase and/or at least one xylanase.

4. The bread according to any of the preceding claims wherein the wheat flour comprises a whole wheat flour.

5. The bread according to any of the preceding claims wherein the heat-stable alpha-amylase is non-GMO enzyme.

6. The bread any of the preceding claims wherein the oats are cut rolled oats.

7. A method of preparing a bread according to any of claims 1 to 6 comprising the steps of
i) providing wheat flour, at least one heat-stable alpha-amylase, wherein the heat-stable alpha-amylase when having an activity of 800 KNU/g is present in the range of from 1 to 3 ppm/kg flour, oats in an amount of from 50 to 250 g per kg flour, and psyllium and/or chia,
ii) forming a dough comprising the ingredients from step i), and
iii) baking said dough from step ii) to form a bread.

8. The method according to claim 7, wherein the baking of step iii) is for at least 4 minutes.

## Patentansprüche

1. Brot umfassend
a) Weizenmehl
b) mindestens eine hitzestabile Alpha-Amylase, wobei die hitzestabile Alpha-Amylase, wenn sie eine Aktivität von 800 KNU/g aufweist, im Bereich von 1 bis 3 ppm/kg Mehl vorliegt, und
c) Hafer in einer Menge im Bereich von 50 bis 250 g pro kg Mehl, und
d) mindestens ein weiteres hydrokolloidales Nicht-Stärke-Pflanzenpolysaccharid, ausgewählt aus der Gruppe bestehend aus Psyllium, Chia und Kombinationen dieser.

2. Brot nach Anspruch 1, wobei das mindestens eine weitere hydrokolloidale Nicht-Stärke-Pflanzenpolysaccharid Psyllium ist.

3. Brot nach einem der voranstehenden Ansprüche, wobei das Brot außerdem mindestens eine nicht hitzestabile Alpha-Amylase und/oder mindestens eine Xylanase enthält.

4. Brot nach einem der voranstehenden Ansprüche, wobei das Weizenmehl ein Weizenvollkornmehl umfasst.

5. Brot nach einem der voranstehenden Ansprüche, wobei die hitzestabile Alpha-Amylase ein Nicht-GVO-Enzym ist.

6. Brot nach einem der voranstehenden Ansprüche, wobei der Hafer geschnittene Haferflocken ist.

7. Verfahren zum Herstellen eines Brotes nach einem der Ansprüche 1 bis 6, umfassend die Schritte
i) Bereitstellen von Weizenmehl, mindestens einer hitzestabilen Alpha-Amylase, wobei die hitzestabile Alpha-Amylase, wenn sie eine Aktivität von 800 KNU/g aufweist, im Bereich von 1 bis 3 ppm/kg Mehl vorhanden ist, Hafer in einer Menge von 50 bis 250 g pro kg Mehl, und Psyllium und/oder Chia,
ii) Formen eines Teigs, der die Zutaten aus Schritt i) umfasst, und
iii) Backen des Teigs aus Schritt ii), um ein Brot zu bilden.

8. Verfahren nach Anspruch 7, wobei das Backen von Schritt iii) mindestens 4 Minuten dauert.

## Revendications

1. Pain comprenant
a) de la farine de blé
b) au moins une alpha-amylase thermostable, l'alpha-amylase thermostable lorsqu'elle a une activité de 800 KNU/g étant présente dans la plage comprise entre 1 et 3 ppm/kg de farine, et
c) de l'avoine en une quantité comprise dans la plage allant de 50 à 250 g par kg de farine, et
d) au moins un polysaccharide végétal hydrocolloïdal supplémentaire non amylacé choisi dans le groupe constitué par ceux de psyllium, chia et des associations de ceux-ci.

2. Pain selon la revendication 1, dans lequel ledit au moins un polysaccharide végétal hydrocolloïdal supplémentaire non amylacé est celui de psyllium.

3. Pain selon l'une quelconque des revendications précédentes, dans lequel ledit pain comprend en outre au moins une alpha-amylase non thermostable et/ou au moins une xylanase.

4. Pain selon l'une quelconque des revendications précédentes, dans lequel la farine de blé comprend une farine de blé complète.

5. Pain selon l'une quelconque des revendications précédentes, dans lequel l'alpha-amylase thermostable est une enzyme non-OGM.

6. Pain selon l'une quelconque des revendications précédentes, dans lequel l'avoine est de l'avoine coupée-roulée.

7. Procédé de fabrication d'un pain selon l'une quelconque des revendications 1 à 6, comprenant les étapes consistant à
i) fournir de la farine de blé, au moins une alpha-amylase thermostable, l'alpha-amylase thermostable lorsqu'elle a une activité de 800 KNU/g étant présente dans la plage de 1 à 3 ppm/kg de farine, de l'avoine en une quantité de 50 à 250 g par kg farine, et du psyllium et/ou de la chia,
ii) former une pâte comprenant les ingrédients provenant de l'étape i), et
iii) cuire ladite pâte provenant de l'étape ii) pour former un pain.

8. Procédé selon la revendication 7, dans lequel la cuisson de l'étape iii) est effectuée pendant au moins 4 minutes.
